# EUROPEAN PATENT APPLICATION

(11) **EP 4 056 626 A1**
(43) Date of publication of application: **14.09.2022**
(21) Application number: 20885767.2
(22) Date of filing: 06.11.2020
(51) Int. Cl.: C08G 65/333, C08G 65/334, C08G 81/00, A61K 9/00, A61K 47/30, A61K 48/00, A61K 41/00, A61P 25/28

(54) **EFFECTIVE INTRANASAL DELIVERY TO BRAIN**

(30) Priority: 07.11.2019 KR 20190141988
(71) Applicant: Industry - University Cooperation Foundation Hanyang University, Seoul 04763 (KR); MASSACHUSETTS INSTITUTE OF TECHNOLOGY, Cambridge, MA 02139 (US)
(72) Inventor: YUN, Chae Ok, Seoul 06583 (KR); LEE, Soo Hwan, Bucheon-si Gyeonggi-do 14590 (KR); KASALA, Dayananda, Seoul 04761 (KR); LANGER, Robert S., Newton, Massachusetts 02459-1363 (US)
(74) Representative: dompatent von Kreisler Selting Werner - Partnerschaft von Patent- und Rechtsanwälten mbB
(86) International application number: PCT/KR2020/015459
(87) International publication number: WO 2021/091281

(57) **Abstract**

The present invention relates to a technique for effective intranasal delivery to the brain. More specifically, the present invention is used for diagnosing, preventing or treating central nervous system encephalopathy, neurodegenerative diseases or brain tumor by effectively delivering to the brain a pH-responsive and bioreducible PPA polymer, which can be used as a drug carrier, by means of nasal administration.

## Description

### [Technical Field]

The present invention relates to intranasal delivery to brain, which is used to diagnose, prevent or treat a central nervous system disease, a neurodegenerative disease or a brain tumor by effectively delivering a pH-sensitive and bioreducible polymer that can be used as a drug delivery system to the brain through intranasal administration.

### [Background Art]

Real-time and non-invasive imaging of disease sites is very desirable for the treatment of a central nervous system disease or a brain tumor. For example, non-invasive tracking and imaging of disease tissue, which is a routinely performed diagnostic procedure, may allow early detection and induce timely intervention in a disease and overall improved prognosis of a patient.

As a novel delivery system capable of bypassing a blood-brain barrier which is the largest barrier in the delivery of a therapeutic agent for treating a central nervous system disease, intranasal delivery comes into the limelight.

A variety of materials have been developed to enhance delivery efficiency to the brain through intranasal administration, and the present invention utilizes a polymer as a carrier.

### [Disclosure]

### [Technical Problem]

The present invention is directed to providing a pH-sensitive and bioreducible polymer capable of being used as a drug delivery system and a pharmaceutical use thereof.

### [Technical Solution]

To achieve the above-described object, the present invention provides a polymer represented by Formula 1 below: 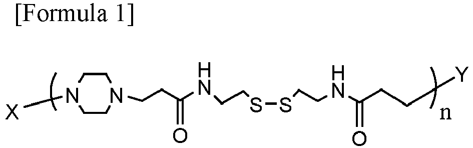 X is PEG having a molecular weight of 2,000-10,000 Da, Y is 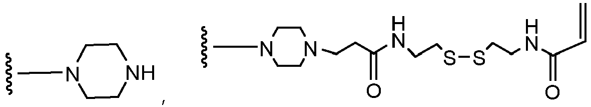 or arginine-conjugated PEI(polyethyleneimine) wherein PEI is branched PEI, and
n is 1 to 10.

The present invention also provides a composition for drug delivery to the brain through nasal route comprising the polymer represented by Formula 1 as a carrier for drug delivery.

The present invention also provides a drug delivery system, which includes the polymer represented by Formula 1; and one or more drugs selected from the group consisting of therapeutic agents and diagnostic agents.

The present invention also provides a method of delivering a drug to the brain through nasal route, which includes intranasally administering the drug delivery system to a subject.

The present invention also provides a method of treating any one of a central nervous system disease, a neurodegenerative disease and a brain tumor, which includes intranasally administering a pharmaceutically effective amount of the drug delivery system to a subject in need thereof.

### [Advantageous Effects]

A pH-sensitive and bioreducible polymer of the present invention can bypass a blood-brain barrier through intranasal administration and improve the bioavailability of a drug, thereby reducing a dose of the drug, and therefore, side effects can be reduced.

In addition, the intranasally-administered drug is delivered to a target disease site of the brain without initially passing through the bloodstream for systemic circulation, resulting in minimizing adverse effects caused by overdosing. In addition, the present invention can effectively deliver a drug to several parts of brain tissue, so that it is suitable for treatment of a central nervous system disease, a neurodegenerative disease or a brain tumor. In addition, the pH-sensitive and bioreducible polymer of the present invention is labeled with a diagnostic labelling material and delivered to brain tissue through intranasal administration, and therefore it can be used in diagnosis of a central nervous system disease, a neurodegenerative disease or a brain tumor.

### [Brief Description of Drawings]

FIG. 1 shows *in vivo* fluorescence images of Ad/PPA-IR780.
FIG. 2 shows the potent antitumor effect of an Ad/PPA-IR780 complex on orthotopic glioblastoma.
FIG. 3 shows the *ex vivo* IVIS imaging results of various organs and *ex vivo* NIR and optical imaging of brain tissue.
FIG. 4 shows the antitumor efficacy of various Ad/polymer complexes.
FIG. 5 shows the biodistribution of various Ad/polymer complexes.
FIG. 6 shows the antitumor efficacy of various Ad/polymer complexes in an orthotopic brain tumor using bioluminescence imaging.
FIG. 7 shows the antitumor efficacy of various Ad/polymer complexes.
FIG. 8 shows the antitumor efficacy of Ad/PPA-IR780 according to various administration routes.
FIG. 9 shows the potent therapeutic efficacy of oAd/PPA-IR780.
FIG. 10 shows the potent therapeutic efficacy of oAd/PPA-IR780.
FIG. 11 shows the PTX concentration in various regions of brain tissues after intranasal or intravenous administration of PPA-PTX-IR780 in a brain tumor-bearing mouse model.
FIG. 12 shows the PTX concentration in various regions of brain tissues after intranasal or intravenous administration of PPA-PTX-IR780 in a normal mouse model.
FIG. 13 shows the bioavailability of PTX in the brain of a tumor-bearing mouse model or normal mouse.
FIG. 14 shows the siRNA distribution profiles in different regions of the brain after intranasal administration of siRNA-Cy5.5/PPA-IR780 in a glioblastoma-bearing mouse model.
FIG. 15 shows the pDNA intensity-time profiles after intranasal or intravenous administration of pDNA-FITC/PPA-IR780 in a brain tumor-bearing mouse model.
FIG. 16 shows the pDNA intensity-time profiles after intranasal or intravenous administration of pDNA-FITC/PPA-IR780 in a normal mouse model.
FIG. 17 shows the bioavailability of pDNA in the brain of a tumor-bearing mouse model or normal mouse model.
FIG. 18 shows the pDNA distribution profiles in different regions of the brain after intranasal administration of a pDNA-FITC/polymer complex in a normal mouse model.
FIG. 19 shows the PET/CT imaging results of the effective delivery of an oAd/PPA-Cu-64-DOTA-herceptine complex through intranasal administration.
FIG. 20 shows the PPA-Erbitux delivery efficiency due to intranasal administration in a normal mouse.
FIG. 21 shows the bioavailability of PPA-IR780-PTX after intranasal administration.
FIG. 22 shows the MR imaging results of the potent antitumor efficacy of PPA-PTX through intranasal administration.
FIG. 23 shows the H&E staining results showing the potent antitumor efficacy of PPA-PTX through intranasal administration in an U87MG/Fluc orthotopic brain tumor model.
FIG. 24 shows the potent antitumor efficacy of PPA-PTX through intranasal administration in an U87MG/Fluc orthotopic brain tumor model.

### [Modes of the Invention]

Hereinafter, the configuration of the present invention will be described in detail.

The present invention relates to a polymer represented by Formula 1 below.

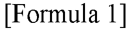

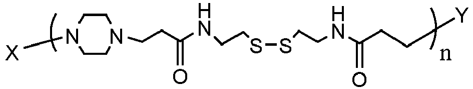

Wherein, X is PEG having a molecular weight of 2,000-10,000 Da Y is 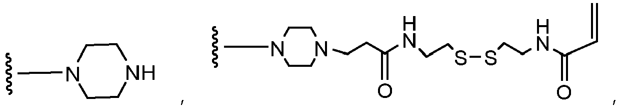 or arginine-conjugated PEI (polyethyleneimine), wherein PEI is branched PEI.
n is 1 to 10.
Y may be further modified so as to have primary amine group. Amine group can be used for conjugation of drug or targeting.

The polymer of Formula 1 of the present invention is uptaken with a high absorption rate in the intracellular pH range (about pH 6.0), and, as a biodegradable and pH-sensitive compound targeting a low pH and hypoxic tumor microenvironment, consists of (i) an escapable portion from immune reactions, (ii) a chargeable portion and (iii) a bioreducible portion having disulfide bonding.

More specifically, the polymer of Formula 1 may be the so-called PP or PPA. If Y is 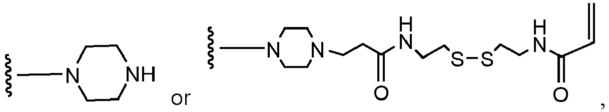 the polymer of Formula 1 represents PP, that is, PEG-Pip-N,N'-cystamine bisacrylamide(CBA). In this specification, PP is also called as PPCBA. In the following examples, PP is also represented as PP5, which means PP using 5 kDa PEG. If Y is arginine-conjugated PEI, the polymer of Formula 1 represents PPA which is synthesized by the reaction of PEG-Pip-CBA (PP) and arginine-conjugated PEI (PEI-Arg). Herein, PEI may be a branched PEI having a molecular weight of 1,000-10,000 Da and arginines may be conjugated to primary amines of PEI. Y may be further modified so as to have primary amine group. Amine group can be used for conjugation of drug or targeting. In the following examples, PPA is also represented as PPA2 or PPA5, which means PPA using 2 kDa or 5 kDa PEG, respectively.

In Formula 1, PEG serves as an escapable portion from immune reactions, and arginine or an amine group is a chargeable portion providing a positive charge to the polymer. Arginine or an amine group provides a positive charge to the polymer, so that it is coupled with a negatively-charged surface (e.g., a negatively-charged surface of an adenovirus) through an ionic interaction, thereby forming a complex. N, N'-cystamine bisacrylamide (CBA) is a moiety serving as a bioreducible portion having disulfide bonding, and a moiety providing pH sensitivity of the polymer is piperazine (Pip).

For example, the synthesis process of PPA will be briefly described as follows:
First, to synthesize PEI-Arg, arginine and polyethyleneimine are conjugated through an EDC/NHS coupling reaction, but not limited thereto.

PEG-Pip-CBA (PP) is synthesized by a reaction of PEG-acrylate, N, N'-cystamine bisacrylamide (CBA) and piperazine (Pip), and then the PEI-Arg is added, thereby synthesizing PPCBA-PEI-Arg (PPA). Herein, PEG-acrylate includes various activated PEG-acrylates, for example, methoxy-, ortho pyridyl disulfide (OPSS), COOH-, NH₂-, or maleimide-PEG acylate could be used. In an embodiment, PEG-acrylate may be methoxy-PEG-acrylate. Alternatively, for PEGylation, other PEG derivatives can be used instead of PEG-acrylate.

Most specifically, a polymer of the present invention is represented by Formula 2.

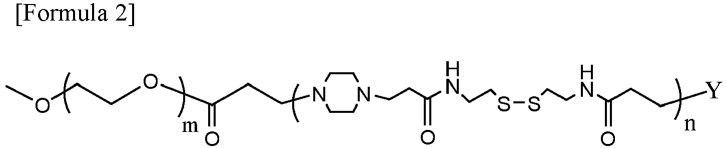

Wherein, Y is 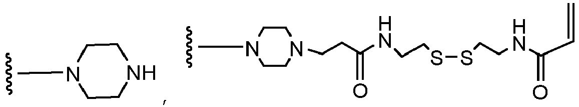 or arginine-conjugated PEI wherein PEI is branched PEI.
n is 1 to 10
m is 44 to 228 (PEG having a molecular weight of 2,000-10,000 Da)
Y may be further modified so as to have primary amine group. Amine group can be used for conjugation of drug or targeting moieties.

The PPA polymer of the present invention is pH-sensitive, and particularly, targets a hypoxic tumor cell microenvironment, and may be used as a drug delivery system for delivering a drug to tumor cells. Alternatively, due to the positively-charged surface, the PPA polymer of the present invention is conjugated with a target component having a negatively-charged surface, and thus can be used as a drug delivery system delivering a drug into the body. Therefore, the PPA polymer may be used as a drug delivery system for delivering a drug to a target disease site, other than tumor cells.

The pH-sensitive polymer of the present invention may be uptaken into brain tissue by bypassing a brain-blood barrier through intranasal administration, and thus may be used as a carrier for drug delivery.

Therefore, in still another aspect, the present invention provides a composition for drug delivery to the brain through nasal route comprising the polymer represented by Formula 1 as a carrier for drug delivery.

In still another aspect, the present invention provides a drug delivery system, which includes the polymer represented by Formula 1; and one or more drugs selected from the group consisting of a therapeutic agent and a diagnostic agent.

The PPA polymer of the present invention may be used as a carrier for drug delivery, or targets a tumor cell microenvironment, or delivers a drug to a target disease site, other than tumor cells via conjugating it with a target component, or may be conjugated with a photosensitizer and delivered to tumor cells, and may kill tumor cells through near-infrared photothermal therapy in the wavelength range of 700 to 900 nm.

The PPA polymer of the present invention may be conjugated with a diagnostic agent on the surface thereof and bypass a brain-blood barrier through intranasal administration to be uptaken into brain tissue, and therefore can be used in diagnosis of a brain disease.

The drug delivery system is preferably a drug delivery system for intranasal administration. The PPA polymer of the present invention may be uptaken into brain tissue by bypassing the brain-blood barrier through intranasal administration, thereby effectively delivering the drug.

The drug delivery system of the present invention may be included in the form of a complex of the PPA polymer and the drug.

The therapeutic agent may be one or more selected from the group consisting of a gene delivery system, a photosensitizer and a pharmaceutically active component.

The gene delivery system may be present in the form of (i) a naked recombinant DNA molecule, (ii) a plasmid, (iii) a viral vector, and (iv) a liposome or noisome containing the naked recombinant DNA molecule or plasmid.

All gene delivery systems used in general gene therapy may be applied, and preferably, a plasmid; a viral vector such as adenoviruses (Lockett LJ, et al., Clin. Cancer Res. 3:2075-2080(1997)), adeno-associated viruses (AAVs, Lashford LS., et al., Gene Therapy Technologies, Applications and Regulations Ed. A. Meager, 1999), retroviruses (Gunzburg WH, et al., Retroviral vectors. Gene Therapy Technologies, Applications and Regulations Ed. A. Meager, 1999), lentiviruses (Wang G. et al., J. Clin. Invest. 104(11):R55-62(1999)), herpes simplex viruses (Chamber R., et al., Proc. Natl. Acad. Sci USA 92:1411-1415(1995)), vaccinia viruses (Puhlmann M. et al., Human Gene Therapy 10:649-657(1999)), reoviruses, poxviruses, the Semliki forest virus, or the measles virus; or a liposome or noisome containing the naked recombinant DNA molecule or plasmid (Methods in Molecular Biology, Vol 199, S.C. Basu and M. Basu (Eds.), Human Press 2002) may be used.

### i. Adenovirus

An adenovirus is widely used as gene delivery vectors due to a medium-sized genome, easy manipulation, a high titer, a wide range of target cells and excellent infectibility. Each of ends of the genome includes an inverted terminal repeat (ITR) of 100 to 200 bp, which is a cis element required for DNA replication and packaging. E1 regions of the genome (E1A and E1B) encode proteins for regulating transcription and transcription of a gene of host cells. E2 regions (E2A and E2B) encode proteins involved in the replication of viral DNA.

Among currently developed adenoviral vectors, an E1 region-deficient replication-incompetent adenovirus is widely used. Meanwhile, an E3 region is removed from a conventional adenoviral vector to provide a site into which a foreign gene is inserted (Thimmappaya, B. et al., Cell, 31:543-551(1982); and Riordan, J. R. et al., Science, 245:1066-1073(1989)). Therefore, a target nucleotide sequence to be delivered into cells is preferably inserted into a deleted E1 region (E1A region and/or E1B region, and preferably, E1B region) or an E3 region, and more preferably, a deleted E1 region. The term "deletion" used in relation to a viral genome sequence, as used herein, includes complete deletion of a corresponding sequence, and partial deletion of the sequence.

An adenovirus has 42 distinct serotypes and A-F subgroups. Among these, adenovirus type 2 and type 5 belonging to subgroup C are the most preferable starting materials for obtaining an adenoviral vector of the present invention. Biochemical and genetic information for adenovirus type 2 and type 5 are well known.

The foreign gene delivered by an adenovirus is replicated in the same manner as an episome, and has very low genetic toxicity against host cells. Therefore, gene therapy using the adenovirus gene delivery system of the present invention is considered to be very safe.

### ii. Retrovirus

A retrovirus is widely used as a gene delivery vector since its genes can be inserted into a host genome, deliver a large amount of foreign genetic materials, and have a wide spectrum of cells capable of being infected.

To construct a retroviral vector, a target nucleotide sequence to be delivered into cells is inserted into a retroviral genome instead of a retroviral sequence, thereby producing a replication-incompetent virus. To produce a virion, a packaging cell line including gag, pol and env genes but not having a long terminal repeat (LTR) and a Ψ sequence is constructed (Mann et al., Cell, 33:153-159(1983)). When a recombinant plasmid including a target nucleotide sequence to be delivered, the LTR and Ψ sequence is introduced into the cell line, the Ψ sequence facilitates the production of an RNA transcriptome of a recombinant plasmid, the transcriptome is packaged within a virus, and the virus is released into a culture medium (Nicolas and Rubinstein "Retroviral vectors," In: Vectors: A survey of molecular cloning vectors and their uses, Rodriguez and Denhardt (eds.), Stoneham: Butterworth, 494-513(1988)). The recombinant retrovirus-containing culture medium is collected and concentrated so as to be used as a gene delivery system.

Gene delivery using a second-generation retroviral vector had been suggested (Kasahara et al. Science, 266:1373-1376 (1994)), and a variant of the Moloney Murine Leukemia Virus (MMLV) was prepared. Here, an erythropoietin (EPO) sequence was inserted into an envelope portion, thereby producing a chimeric protein having a novel binding characteristic. The gene delivery system of the present invention may also be prepared according to a strategy of constructing the second-generation retroviral vector.

### iii. AAV vector

Since an adeno-associated virus (AAV) may infect undivided cells and have an ability to be infected into various types of cells, it is suitable as a gene delivery system of the present invention. Detailed description of the preparation and uses of the AAV vector is disclosed in detail in U.S. Patent Nos. 5,139,941 and 4,797,368.

Research on AAV as a gene delivery system has been disclosed in LaFace et al, Virology, 162:483486 (1988), Zhou et al., Exp. Hematol. (NY), 21:928-933 (1993), Walsh et al, J. Clin. Invest., 94:1440-1448 (1994) and Flotte et al., Gene Therapy, 2:29-37 (1995).

Typically, the AAV is prepared through co-transformation with a plasmid including a target gene sequence (target nucleotide sequence to be delivered into cells) located beside two AAV terminal repeats (McLaughlin et al., J. Virol., 62:1963-1973(1988); and Samulski et al., J. Virol., 63:3822-3828(1989)), and an expression plasmid including a wild-type AAV coding sequence without a terminal repeat (McCarty et al., J. Virol., 65:2936-2945( 1991)).

### iv. Other viral vectors

Other viral vectors may also be used as the gene delivery system of the present invention. Vectors derived from vaccinia viruses (Puhlmann M. et al., Human Gene Therapy 10:649-657(1999); Ridgeway, "Mammalian expression vectors," In: Vectors: A survey of molecular cloning vectors and their uses. Rodriguez and Denhardt, eds. Stoneham: Butterworth, 467-492(1988); Baichwal and Sugden, "Vectors for gene transfer derived from animal DNA viruses: Transient and stable expression of transferred genes," In: Kucherlapati R, ed. Gene transfer. New York: Plenum Press, 117-148(1986) and Coupar et al., Gene, 68:1-10(1988)), lentiviruses (Wang G. et al., J. Clin. Invest. 104(11):R55-62(1999)) and herpes simplex viruses (Chamber R., et al., Proc. Natl. Acad. Sci USA 92:1411-1415(1995)) may also be used as a delivery system capable of delivering a target nucleotide sequence into cells.

Other than this, as viral vectors, reoviruses, poxviruses, the Semliki forest virus and the measles virus are used.

### v. Liposome

A liposome is automatically formed by phospholipids dispersed in an aqueous phase. Examples of successful delivery of a foreign DNA molecule into cells using liposomes are disclosed in Nicolau and Sene, Biochim. Biophys. Acta, 721:185-190 (1982) and Nicolau et al., Methods Enzymol., 149:157-176 (1987). Meanwhile, as the most widely used reagent for transformation of animal cells using a liposome, Lipofectamine (Gibco BRL) is used. The liposome containing the target nucleotide sequence to be delivered is interacted with a cell through a mechanism such as endocytosis, adsorption to the cell surface or fusion with a plasma cell membrane to deliver the target nucleotide sequence into cells.

In one embodiment of the present invention, the gene delivery system may be a recombinant adenovirus.

As many advantages of a recombinant adenovirus as a gene delivery vector are manifested, the frequency of its use in cancer gene therapy is steadily increasing. Particularly, when cancer is treated with a gene therapeutic agent, there is an advantage of no need of long-term and long-lasting expression of a therapeutic gene. In addition, since the host's immune response induced by a virus used as a vector is not a significant problem or can be an advantage, a recombinant adenovirus is in the spotlight as a gene carrier for cancer therapy.

The recombinant adenovirus may be a replication-incompetent adenovirus or oncolytic adenovirus.

The replication-incompetent adenovirus is recombined by inserting a therapeutic gene instead of (a total or part of) an E1 gene required for replication of the adenovirus to prevent replication in adenovirus-introduced cells.

The oncolytic adenovirus is an adenovirus from which E1B-55 kDa gene is partially deleted, and can be proliferated only in p53-functionally inactivated cells. While viral replication is actively performed in p53 function-suppressed cancer cells, viral replication is inhibited in normal cells. Therefore, the oncolytic adenovirus does not affect normal cells, and selectively kills cancer cells. For this reason, it is particularly advantageous for cancer treatment.

In one embodiment of the present invention, a recombinant adenovirus has an inactivated E1B-19 kDa gene, an E1B-55 kDa gene or an E1B-19 kDa/E1B-55 kDa gene, and preferably has an inactivated E1B-19 kDa and an E1B-55 kDa gene.

The term "inactivation" in relation to a gene means that functions of a normal protein encoded by the gene, as used herein, are not exhibited because the transcription and/or translation of the gene are not normally performed. For example, the inactivated E1B-19 kDa gene is a gene in which a mutation (substitution, addition, partial deletion or total deletion) occurs, and does not produce an activated E1B-19 kDa protein. When the E1B-19 kDa gene is absent, a cell apoptosis ratio may be increased, and when the E1B-55 kDa gene is absent, tumor cell specificity is exhibited (see Korean Patent Application No. 2002-0023760).

According to one embodiment of the present invention, the recombinant adenovirus of the present invention may include an activated E1A gene. The E1A gene-containing recombinant adenovirus has a replication-competent characteristic. According to a more preferable embodiment of the present invention, the recombinant adenovirus of the present invention includes an inactivated E1B-19 kDa/E1B-55 kDa gene and an activated E1A gene.

In one embodiment of the present invention, the gene delivery system may be an E1 region-deleted replication-incompetent tumor-specific oncolytic adenovirus.

In addition, the vector preferably further includes a selection marker. The term "selection marker", as used herein, facilitates selection of cells transformed by introducing an shRNA expression cassette. As a selection marker capable of being used in the vector of the present invention, any gene capable of easily detecting or measuring the introduction of a vector may be used without particular limitation. Typically, markers imparting selectable phenotypes such as drug resistance, auxotrophy, tolerance to cytotoxic agents or expression of surface proteins include, for example, a green fluorescent protein (GFP), puromycin, neomycin (Neo), hygromycin (Hyg), a histidinol dehydrogenase gene (hisD) or guanine phosphoribosyltransferase (Gpt).

In addition, the photosensitizer may be selected from the group consisting of phorphyrins, chlorins, bacteriochlorins, phthalocyanines, naphthalocyanines and 5-aminolevuline esters.

Preferably, IR-780 or Cu-64-DOTA exhibiting a near-infrared photothermal effect in a range of 700 to 900 nm is used.

In addition, the type of a pharmaceutically active component of the present invention is not particularly limited, and may be, for example, one or more selected from the group consisting of an anticancer agent, an antibiotic, a hormone, a hormone antagonist, an interleukin, an interferon, a growth factor, a tumor necrosis factor, an endotoxin, a lymphotoxin, a urokinase, a streptokinase, a tissue plasminogen activator, a protease inhibitor, an alkylphosphocholine, a radioisotope-labeling component, a surfactant, a cardiovascular system drug and a nervous system drug.

Among these, the anticancer agent may be bevacizumab, temozolomide, nitrosourea, cisplatin, procarbazine+CCNU+vincristine (PCV), vinblastine, vincristine, vinflunine, vindesine, vinorelbine, cabazitaxel, docetaxel, larotaxel, ortataxel, paclitaxel, tesetaxel, ixabepilone, herceptin, erbitux, cyclophosphamide, doxorubicin, etoposide, topotecan, carboplatin, procarbazine, mechlorethamine, ifosfamide, melphalan, chlorambucil, bisulfan, dactinomycin, daunorubicin, bleomycin, plicomycin, mitomycin, tamoxifen, transplatinum, 5-fluorouracil or methotrexate.

The drug delivery system of the present invention may be used to prevent or treat a brain tumor due to the tumor targeting property of a PPA polymer, and may deliver a drug to a target disease site by conjugation with a target component, and therefore, it can be used to prevent or treat a central nervous system disease or a neurodegenerative disease.

The central nervous system disease may be a cognitive disorder, intellectual disability, microencephaly, brain metastasis, a neurodevelopmental disorder, dementia, an autistic spectrum disorder, Down's syndrome, Rett syndrome, or fragile X syndrome.

The neurodegenerative disease may be an ischemic stroke, a traumatic brain injury, acute disseminated encephalomyelitis, amyotrophic lateral sclerosis (ALS), retinitis pigmentosa, mild cognitive impairment, Alzheimer's disease, Pick's disease, senile dementia, progressive supranuclear palsy, cortical dementia, Wilson's disease, a multiple infarct disease, arteriosclerotic dementia, AIDS-associated dementia, cerebellar degeneration, spinocerebellar degeneration syndromes, Friedreich's ataxia, ataxia telangiectasia, an epilepsy-associated brain injury, a spinal cord injury, restless legs syndrome, Huntington's disease, Parkinson's disease, striatonigral degeneration, cerebral vasculitis, mitochondrial encephalomyopathies, neuronal ceroid lipofuscinosis, spinal muscular atrophies, a central nervous system-associated lysosomal storage disorder, leukodystrophies, an urea cycle defect disorder, hepatic encephalopathies, renal encephalopathies, metabolic encephalopathies, porphyria, bacterial meningitis, viral meningitis, meningoencephalitis, prion diseases, poisonings with neurotoxic compounds, Guillain Barre syndrome, chronic inflammatory neuropathies, polymyositis, dermatomyositis or radiation-induced brain damage.

The brain tumor or encephaloma refers to a tumor occurring in brain matter and meninges. The brain tumor may be glioma, oligodendroglioma, glioblastoma, colloid cyst, epidermoid cyst meningioma, hemangioblastoma, lymphoma, pituitary adenoma, a metastatic tumor, or a combination thereof. For example, the glioma is glioblastoma multiforme (GBM). The brain tumor may be a primary brain tumor or a tumor metastasized from a different cancer.

In addition, the PPA polymer of the present invention may be labeled with a diagnostic agent (diagnostic labelling material), and the polymer labeled with the labelling material may be traced *in vivo,* and therefore, optical detection and imaging of a central nervous system disease, a neurodegenerative disease or a brain tumor are possible.

The diagnostic agent may be any material that can allow a target cell to be detectable without limitation. For example, the diagnostic agent may be a near-infrared fluorescent material capable of transmitting an image of a living body, such as cyanine, allophycocyanin, fluorescein, tetramethylrhodamine, BODIPY or Alexa; a radiopharmaceutical such as Calcium-47, Carbon-11, Carbon-14, Chromium-51, Cobalt-57, Cobalt-58, Erbium-169, Fluorine-18, Gallium-67, Gallium-68, Hydrogen-3, Indium-Ill, Iodine-123, Iodine-131 or Technetium-99m; or an MRI contrast agent.

The drug delivery system of the present invention may be administered to tissue or cells isolated from a subject to be diagnosed so as to allow the PPA polymer and/or the diagnostic agent to be sensed as a signal and thus obtain an image. To sense the signal, a magnetic resonance imaging (MRI) device or optical imaging device may be used. The MRI device is for generating images by putting a living body in a strong magnetic field, irradiating radio waves of a specific frequency to allow absorption of energy into an atomic nucleus such as hydrogen present in body tissue and make a high energy state, interrupting the radio waves to emit the atomic nucleus energy such as hydrogen and convert this energy to a signal, and processing the signal with a computer. Since the magnetic or radio waves are not disturbed by bones, clear three-dimensional tomographic images for tumors in the peripheral region of the rigid bone, brain or bone marrow can be obtained in longitudinal and transversal directions and at any angles. Particularly, the MRI device is preferably a T2 spin-spin relaxation MRI device.

The drug delivery system of the present invention may further include a pharmaceutically acceptable carrier, and may be formulated with the carrier. The term "pharmaceutically acceptable carrier", as used herein, refers to a carrier or diluent which does not irritate an organism and does not suppress the biological activity and property of an administered compound. As an acceptable pharmaceutical carrier for a drug delivery system formulated in a liquid solution, which is suitable for sterilization and a living body, saline, sterile water, Ringer's solution, buffered saline, an albumin injection, a dextrose solution, a maltodextrin solution, glycerol, ethanol, and a mixture of one or more components thereof may be used, and if needed, other conventional additives such as an antioxidant, a buffer, a bacteriostatic agent, etc. may be added. In addition, the drug delivery system may be formulated as an injectable form such as an aqueous solution, a suspension or an emulsion, a pill, a capsule, a granule or a tablet by additionally adding a diluent, a dispersing agent, a surfactant, a binder, a lubricant, etc.

As a formulation for intranasal administration, which includes the drug delivery system of the present invention as an active ingredient, there is an injectable type, or a spray type such as an aerosol capable of being inhaled through a respirator. As an injectable formulation, the drug delivery system of the present invention may be formulated in a solution or suspension by being mixed with a stabilizer or buffer in water, and prepared for unit administration as an ampoule or vial. To be formulated in a spray type such as an aerosol, a propellant may be mixed with the above-mentioned additives so as to disperse a water-dispersed concentrate or wet powder.

The drug delivery system of the present invention may be sprayed into nasal cavity-brain administration route using a drug delivery device for nasal cavity to brain.

As the drug delivery device for nasal cavity to brain, a conventional nebulizer type may be used.

The drug delivery system of the present invention may be used as a single therapy, or may be used in combination with a different conventional therapy such as chemotherapy or radiation therapy. When such combined therapy is performed, a more effective disease treatment is possible.

Therefore, in still another aspect, the present invention provides a method of delivering a drug to the brain through nasal route, which includes intranasally administering the drug delivery system to a subject.

In still another aspect, the present invention provides a method of treating any one of a central nervous system disease, a neurodegenerative disease or a brain tumor, which includes intranasally administering a pharmaceutically effective amount of the drug delivery system to a subject in need thereof.

The term "pharmaceutically effective amount", as used herein, refers to an amount sufficient for treating a disease at a reasonable benefit/risk ratio applicable for medical treatment. A suitable dose of the drug delivery system of the present invention varies according to a formulation method, an administration method, a patient's age, weight and sex, the severity of a disease symptom, a diet, an administration time, an excretion rate and reaction sensitivity, and an ordinarily skilled doctor may easily determine and prescribe a dosage effective for desired treatment. Generally, the drug delivery system of the present invention includes a complex of 1×10⁵ to 1×10¹⁵ pfu/mL of a polymer or polymer-gene delivery system, a photosensitizer, and a pharmaceutically active component, and conventionally injected at 1×10¹⁰ pfu every other day for 2 weeks.

The term "subject", as used herein, includes an animal such as a horse, sheep, a pig, a goat, a camel, an antelope or a dog, or a human, which has a disease that can be improved by administration of the drug delivery system of the present invention. By administering the drug delivery system according to the present invention to a subject, the disease may be effectively prevented or treated. The treatment method according to the present invention may be a method of treating an animal excluding a human, but the present invention is not limited thereto. On the other hand, in the case of a human, considering that a disease is capable of being improved in symptoms by administration of the drug delivery system according to the present invention, the drug delivery system of the present invention may be sufficiently used in human treatment.

### [Examples]

Hereinafter, experimental examples of the present invention will be described in detail. The following experimental examples are merely provided to explain the present invention, but the scope of the present invention is not limited to the following experimental examples. The experimental examples allow the disclosure of the present invention to be complete, and are provided to completely inform the scope of the present invention to those of ordinary skill in the art to which the present invention belongs. The present invention is only defined by the scope of the claims.

### <Example 1> Synthesis of PPCBA-PEI-Arg

### (1) Synthesis of poly(ethyleneimine)-arginine (PEI-Arg)

Arginine was conjugated to polyethyleneimine according to the references (Journal of Colloid and Interface Science 348 (2010) 360-368, Biomaterials 31(2010) 8759-8769). The carboxyl group of arginine (350 mg, 2.0 mmol) was coupled with an EDC/NHS (EDC, 384 mg, 2.0 mmol and NHS = 230 mg 2.0 mmol) reagent in phosphate saline buffer (PBS, pH= 7.4 (PBS, 3.0 mL)) at 4 °C for 4 hours for activation. Then, polyethylenimine (PEI; 360 mg, 0.2 mmol)) was added to activate the arginine, and the reaction was performed at room temperature for 18 hours. The reaction product was dialyzed against double distilled water for one day (MWCO 1.0 kDa) to remove an unreacted compound and freeze-dried, thereby obtaining a white material, PEI-Arg. A chemical structure was identified by ¹H NMR (600 MHz, D₂O). A characteristic peak of PEI (2.0 to 3.0 ppm) and arginine peaks (1.66 (-HCCH₂CH₂CH₂NH-); 1.86 (-HCCH₂CH₂CH₂NH-); 3.24 (-HCCH₂CH₂CH₂NH-); 3.86 (-HCCH₂CH₂CH₂NH-)) were confirmed.

### (2) Synthesis of PPCBA-PEI-Arg (PPA)

To produce a pH-sensitive and bioreducible polymer, mPEG-acrylate (125 mg, 0.025 mM (PEG = 5.0 kDa or 2.0 kDa; Laysan Bio, Inc)) and N, N'-cystamine bisacrylamide (71.5 mg, 0.275 mM; PolySciences Inc., Warrington, PA) were dissolved in methanol (5.0 mL). Subsequently, piperazine (19.5 mg; Sigma-Aldrich) was added, the reaction mixture was stirred in a 100% nitrogen atmosphere at 50 to 60 °C for 48 hours under a dark condition and cooled at room temperature, and then PEI-Arg (12 mg) was added. The resulting reaction mixture was stirred at room temperature for one day, the final polymer was dialyzed against double distilled water for one day (MWCO 3.5 kDa, Pro Spectrum, dialysis membrane) and freeze-dried, thereby obtaining a white material, PPCBA-PEI-Arg. The chemical structure of PPCBA-PEI-Arg was identified from resonance peaks at 2.3-2.8 ppm and 3.6 ppm corresponding to the -N(CH₂-CH₂) methylene proton of the piperazine and the -S-CH₂-CH₂-NH-CO- proton of CBA, respectively, as well as the characteristic peaks of mPEG and PEI-Arg using ¹H NMR (600 MHz, D₂O):

### (3) Cell line and cell culture

A U87MG-Fluc cell line was purchased from the American Type Culture Collection (ATCC, Manassas, VA), cultured in a DMEM (Gibco BRL, Grand Island, NY) containing 10% FBS (Gibco BRL) and HEPES (Gibco BRL) at 37 °C with 5% CO₂.

### (4) Preparation of adenovirus (Ad)

A GFP-expressing non-replicable Ad (dE1/GFP) and an oncolytic Ad (DWP418, RdB/IL-12/Decorin or HE5cT-Rd19-k35/Decorin; oAd) were used in an E1 region under the control of a CMV promoter, and basically, the method described in the previous research that had conducted by the inventors was used (Kim, E. et al. Hum. Gene Ther. 2003, 14, 14151428; Kim, J. H. et al. J. Natl. Cancer Inst. 2006, 98, 14821493; Choi, J. W. et al. Gene Ther. 2013, 20, 880892; Kim, P. H. et al. Biomaterials 2011, 32, 93289342). All Ads were propagated in HEK293 cells, and purified by a CsCl (Sigma, St Louis, MI) concentration gradient. The number of viral particles (VPs) was calculated based on the assumption that the absorbance of 1 measured at OD₂₆₀ is equivalent to 10¹² VP/mL. Viral infectious titers (PFU/mL) were determined using a limiting dilution assay for HEK293 cells. Viral particle/PFU ratios of dE1/GFP and DWP418 were 29:1 and 81:1, respectively. The MOI was calculated from the virus infectious titers.

### (5) Preparation of Ad/PPA complex

To prepare the Ad/PPA complex, Ad particles (2×10¹⁰ VP/PBS, pH 7.4) were mixed with various concentrations of the PPA polymer. As a result, the molar ratios of the Ad particles per polymer were 1×10⁵ and 1×10⁶. Before use, the solution was incubated at room temperature for 30 minutes.

### (6) Statistical analysis

Data was expressed as mean ± standard deviation (SD). Statistical analysis was performed using a two-tailed Student's t test (SPSS 13.0 software; SPSS, Chicago, IL). A P value less than 0.05 was considered statistically significant.

### <Experimental Example 1> In vivo fluorescence images of Ad/PPA-IR780

To analyze the biodistribution of an Ad/PPA-IR780 complex, the complex was injected intranasally, and 5 minutes, 6, 12, 18, 24 or 48 hours after the injection, NIR imaging was performed.

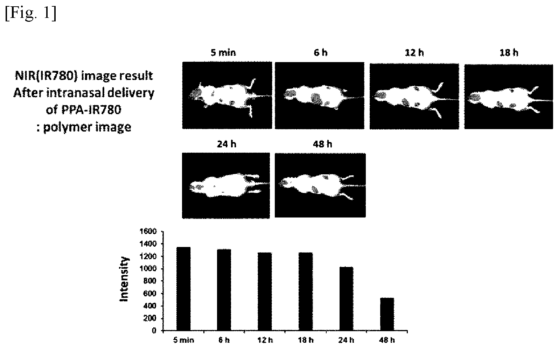

FIG. 1 shows that signals are similarly maintained 5 minutes to until 18 hours after injection, and then gradually reduced in a time-dependent manner up to 48 hours. As the Ad/PPA-IR780 complex was uptaken into tumor tissue, much of a fluorescent signal was maintained in a tumor-bearing brain region: the maximum fluorescence intensity in the tumor region was achieved at 5 minutes after injection. This result suggests that the Ad/PPA-IR780 complex is a suitable therapeutic agent for targeting a brain tumor through an intranasal route.

### <Experimental Example 2> Potent anticancer effect of Ad/PPA-IR780 complex

To construct a brain tumor model, U87MG-Fluc, which is a human glioblastoma cell line stably expressing a firefly luciferase gene, was stereotactically injected into the brain using a Hamilton syringe. To non-invasively monitor tumor growth, bioluminescence imaging was performed at 6-day intervals. On day 21, the Ad/PPA5-IR780 complex was intranasally injected, and then the brain tumor was irradiated with laser at 6 hours after the injection.

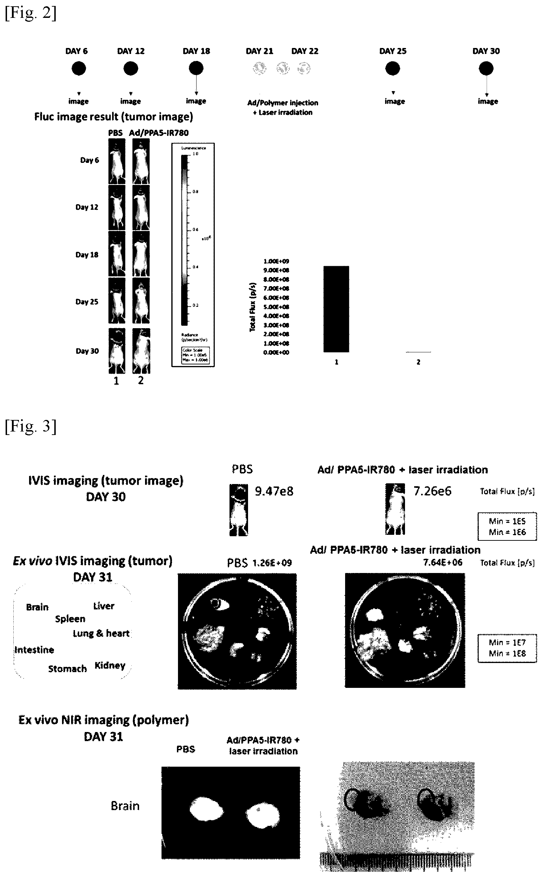

As shown in FIG. 2, at 30 days after the tumor cell injection, the luciferase signal obtained from the orthotopic brain tumors of mice treated with Ad/PPA5-IR780 (7.26×10⁶ p/s) was significantly lower than those observed in a vehicle-treated group, showing 131-fold lower tumor fluorescence intensity than PBS (9.48×10⁸ p/s). This result shows that the Ad/PPA5-IR780 complex is capable of inducing the inhibition of tumor growth of highly aggressive orthotopic glioblastoma.

### <Experimental Example 3> Ex vivo imaging for monitoring tumor targeting and antitumor effects

The fluorescence intensity of orthotopic tumors was assessed by *ex vivo* bioluminescence imaging.

As shown in FIG. 3, the fluorescence intensity was 165-fold lower in the Ad/PPA5-IR780-treated group (7.64×10⁶ p/s), compared with a PBS-treated group (1.26×10⁹ p/s). This result demonstrates the potent antitumor effect of the intranasally-administered Ad/PPA5-IR780 complex.

In addition, the potent antitumor efficacy of the Ad/PPA5-IR780 complex was reconfirmed by the comparison of *ex vivo* NIR imaging and optical images.

### <Experimental Example 4> Antitumor efficacy and biodistribution of Ad/PPA-IR780

A U87MG-Fluc orthotopic glioblastoma model was used to compare the therapeutic efficacy and biodistribution profiles of various intranasally-administered nanocomplexes using various types of IR780-conjugated polymers. All treatments were administered through intranasal administration at 6 days after cell injection. All tumors were irradiated with laser at 6 hours after the intranasal administration of the nanocomplexes.

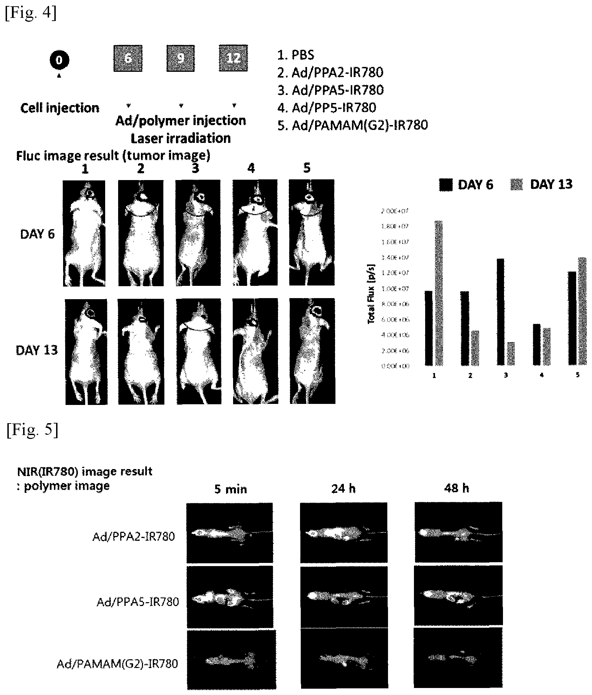

As shown in FIG. 4, at 13 days after tumor cell injection, the luciferase signal from the orthotopic tumors of mice treated with Ad/PPA5-IR780, Ad/PPA2-IR780, Ad/PP-IR780 were attenuated PP

To evaluate biodistribution profiles of various Ad/polymer complexes, NIR imaging was performed every 24 hours after the first intranasal administration of a therapeutic agent at 6 days after tumor cell injection.

As shown in FIG. 5, mice treated with Ad/PPA2-IR780 or Ad/PPA5-IR780 showed a higher fluorescence intensity in brain tissue in comparison with mice treated with Ad/PAMAM(G2)-IR780 (PAMAM is disclosed in PCT/KR/2017/002449); and after the administration of Ad/PAMAM(G2)-IR780, minimal to no fluorescence was detected in a brain region. This result suggests that efficient brain delivery through intranasal injection is a general function of a PPA-based polymer.

### <Experimental Example 5> Antitumor efficacy of Ad/PPA-IR780: bioluminescence imaging

At 6 days after cell injection, the establishment of an orthotopic brain tumor was confirmed through bioluminescence imaging. For this treatment, mice (tumor photon value approached 1×10⁶ p/s) were intranasally injected with 20 µL of PBS, naked Ads (5×10¹⁰ VP), Ad/APP (APP is disclosed in Biomaterials 33 (2012) 8122-8130), Ad/PP5-PTX-Erbitux or Ad/PPA5-IR780 (1×10⁵, 1×10⁶; molar ratios of polymer: Ad) every 3 days for a total of three times. After the intranasal administration, all tumors were irradiated with laser.

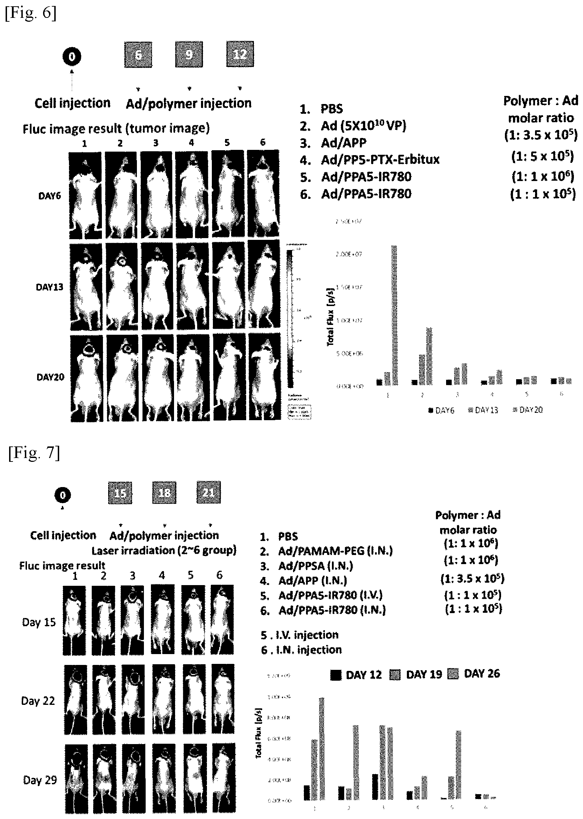

As shown in FIG. 6, at 20 days after tumor cell injection, the luciferase signal obtained from the orthotopic tumors of mice treated with Ad/PPA5-IR780 (molar ratio: 1×10⁵) was more efficiently attenuated in comparison with other groups, respectively showing 3.40-, 2.32- or 1.44-fold higher therapeutic efficacy than that of Ad/APP, Ad/PP5-PTX-Erbitux, or Ad/PPA5-IR780 (molar ratio of 1×10⁶). These results suggest that PP-based pH-sensitive polymers (PP and PPA) can efficiently deliver Ads to the brain through intranasal injection.

### <Experimental Example 6> Antitumor efficacy and different administration routes of Ad/PPA-IR780

To compare the antitumor effects of various Ad/polymer complexes, mice were intranasally administered Ad/PAMAM-PEG, Ad/PPSA (PPSA is disclosed in KR10-2016-0103078), Ad/APP or Ad/PPA5-IR780 at 3-day intervals for a total of three times. A group of mice received Ad/PPA5-IR780 through an intravenous route (named Ad/PPA5-IR780 (I.V.) group). All tumors were irradiated with laser at 6 hours after treatment.

As shown in FIG. 7, at 26 days after tumor cell injection, the luciferase signal obtained from orthotopic tumors of mice treated with Ad/PPA5-IR780 (I.N. injection) was significantly lower than other groups, that is, 30.5-, 29.5-, 9.66-, or 27.9-fold lower tumor fluorescence was shown compared to the group treated with Ad/PAMAM-PEG, Ad/PPSA, Ad/APP or Ad/PPA5-IR780 (I.V. injection), respectively, demonstrating that PPA5-IR780-mediated intranasal delivery induces much better therapeutic efficacy than intravenous delivery.

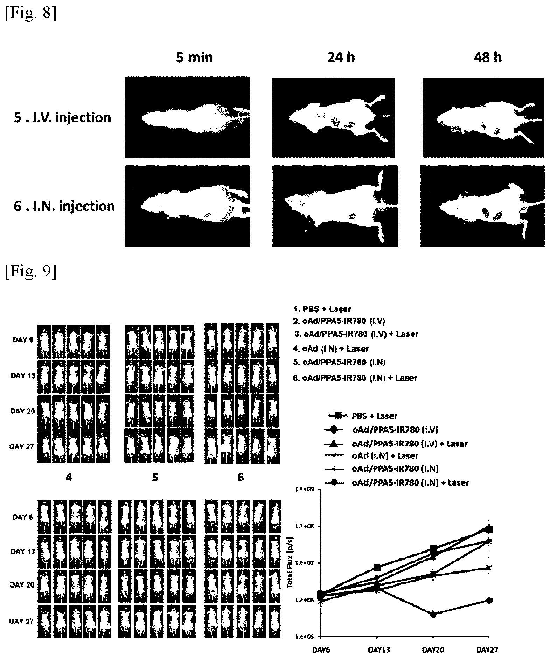

In addition, as shown in FIG. 8, a biodistribution investigation showed fluorescent signals obtained from Ad/PPA5-IR780 (I.N. injection) was detected in the brain during an entire observation period, but when Ad/PPA5-IR780 was intravenously administered, no signal was detected in the brain tissue. This indicates that the intranasal administration is critical for Ad/PPA-IR780 accumulation in a brain tumor and its potent antitumor effect.

### <Experimental Example 7> Potent therapeutic efficacy of oAd/PPA-IR780 in orthotopic brain cancer model

To assess the combined therapeutic efficacy of photothermal therapy by an oncolytic Ad (oAd) and IR780 *in vivo,* oAd/PPA5-IR780 was intravenously (I.V.) injected, or PBS, oAd or oAd/PPA5-IR780 was intranasally (I.N.) injected into luciferase-expressing orthotopic brain tumors. At 24 hours after injection, tumors were irradiated with 808-nm laser for 3 minutes.

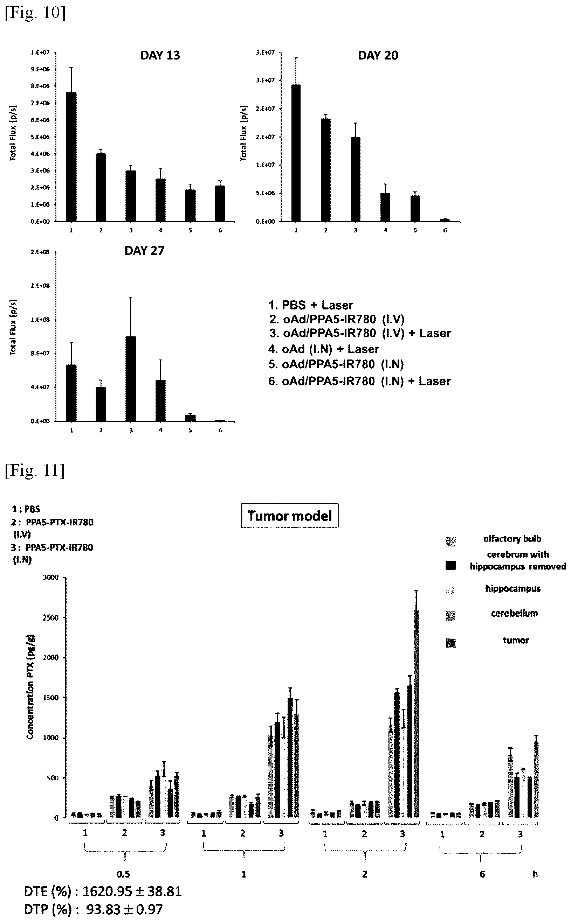

As shown in FIG. 9, compared with other groups, a significantly higher antitumor effect was induced by laser irradiation in combination with intranasal administration of oAd/PPA5-IR780. At 27 days after tumor cell injection, the luciferase signal obtained from the orthotopic brain tumors of mice treated with oAd/PPA5-IR780 (I.N.) in combination with laser irradiation (9.8×10⁵ +/- 2.1×10⁵ p/s) was significantly attenuated compared with other treatment groups (P < 0.01), and showed a 67.5-, 40.8-, 101.8-, 49.2-, or 7.5-fold lower luciferase signal than the results of treatment with laser (6.6×10⁷ +/- 2.7×10⁷ p/s) and PBS, treatment with oAd/PPA5-IR780 (I.V.) without laser (4.0×10⁷ +/- 8.6×10⁶ p/s), treatment with laser (4.8×10⁷ +/- 2.4×10⁷ p/s) and oAd, or treatment with oAd/PPA5-IR780 (I.N.) without laser (7.3×10⁶ +/- 2.1×10⁶ p/s), respectively (FIG. 10). These results demonstrate the potent therapeutic efficacy of oAd/PPA-IR780 with laser treatment after intranasal administration.

### <Experimental Example 8> PTX distribution profile in brain tissue after intranasal administration of PPA-IR780-PTX

To determine whether PTX conjugated to a PPA-IR780 polymer can be efficiently delivered to brain tissue, a PTX-conjugated polymer variant (PPA5-PTX-IR780) was administered through any one of intravenous and intranasal routes. At 0.5, 1, 2 and 6 hours after injection, brain tissues were collected, and PTX amounts in various anatomical regions of the brain tissues were determined by LC-MS/MS.

As shown in FIG. 11, in a brain tumor-bearing mouse model, the PTX amount in the subregion of the brain elevated irregularly within 0.5 hour, and peaked at 2 hours after intranasal injection. At 2 hours after the intranasal injection, the PTX concentration in a tumor was the highest among the subregion of the brain.

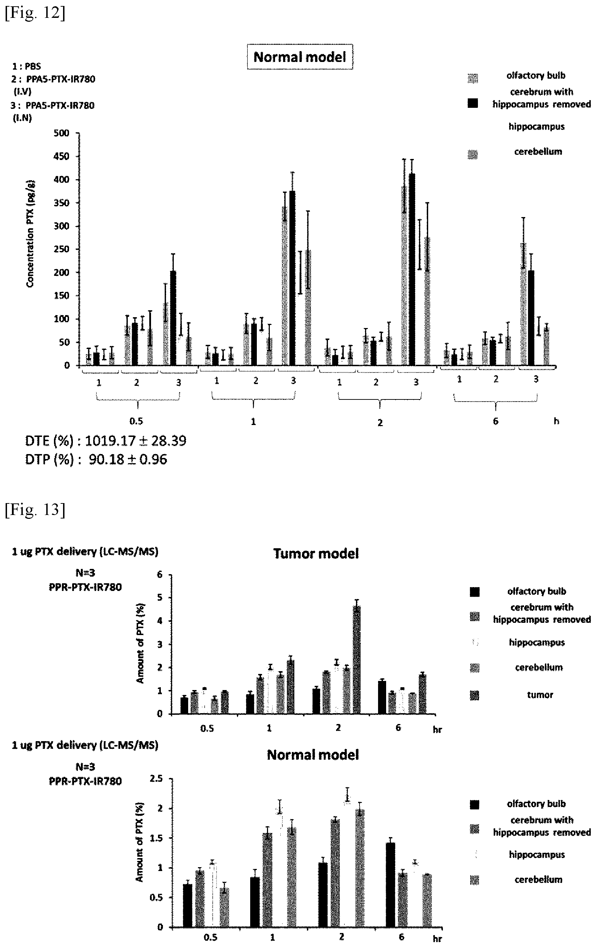

In a normal mouse model, the intranasal injection of PPA5-PTX-IR780 led to efficient uptake of PTX into brain tissues, similar to that observed in tumor-bearing mice in FIG. 11 (FIG. 12); a similar biodistribution pattern was observed in both mice, but the total PTX amount was slightly lower in the normal mice than tumor-bearing mice. These results suggest that the presence of tumors is not necessary for efficient brain delivery of PPA5-PTX-IR780. Importantly, these results suggest that the backbone of the PPA-IR780 polymer can be used as a drug delivery system for treating a central nervous system disease, a neurodegenerative disease or a brain tumor.

After the intranasal delivery of PPA5-PTX-IR780, the efficiency of direct nose-to-brain transport (DTP) of PTX was analyzed by calculating DTP and drug targeting efficiency (DTE) with values obtained from FIGS. 11 and 12. DTP% represents the percentage of a drug delivered directly to the brain through an olfactory route. PPA5-PTX-IR780 showed the highest DTE% (1620.95 +/- 38.81 in a tumor model and 1019.17 +/- 28.39 in a normal model) and DTP% (93.83 +/- 0.97 in a tumor model and 90.18 +/- 0.96 in a normal model), suggesting that PPA5-PTX-IR780 administration through an intranasal route facilitates the delivery of a therapeutic agent to the brain, compared with intravenous injection due to efficient delivery through the olfactory region of the nasal cavity.

As shown in FIG. 13, when the tumor and normal models were compared, the amount of PTX delivered to the brain via the intranasal route was higher in the tumor model.

### <Experimental Example 9> siRNA distribution in brain after intranasal administration of siRNA-Cy5.5/PPA-IR780

To assess whether siRNA can be intranasally delivered by the PPA5-IR780 polymer, Cy5.5-labeled siRNA (siRNA-Cy5.5) was complexed with PPA5-IR780 for intranasal administration. At 0.5, 1, 2 and 6 hours after injection, brain tissues were tested to identify the presence of siRNA-Cy5.5 using a fluorescent reader.

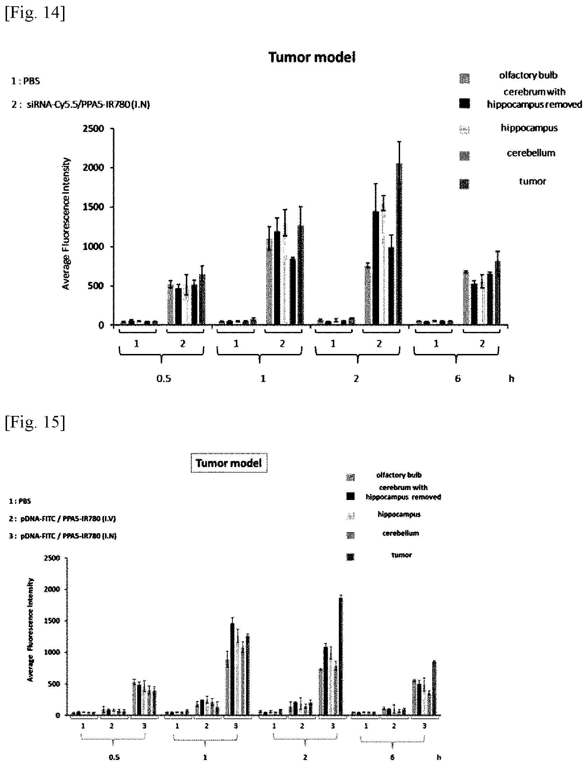

As shown in FIG. 14, the fluorescence intensity of intranasally-administered Cy5.5-labeled siRNA reached the highest level in orthotopic brain tumors two hours after injection; and the intensity at this time in a tumor region was superior to those obtained in all other regions of the brain. These results show that siRNA can also be efficiently delivered to a tumor through an intranasal route using PPA-IR780 as a carrier.

### <Experimental Example 10> pDNA distribution in brain after intranasal administration of pDNA-FITC/PPA-IR780

To determine whether pDNA is intranasally delivered to the brain and assess the delivery efficiency thereof, FITC-labeled pDNA (pDNA-FITC) was complexed with PPA5-IR780 for intranasal delivery. The pDNA-FITC/PPA5-IR780 complex was administered intravenously and intranasally. At 0.5, 1, 2 and 6 hours after injection, brain tissue was tested to confirm the presence of pDNA using a fluorescent reader.

As shown in FIG. 15, intranasally administered FITC-labeled pDNA was observed at a significantly higher level in various parts of the brain compared to intravenous administration. Importantly, the highest level of pDNA was detected in tumor tissue 2 to 6 hours after injection, demonstrating that PPA5-IR780 promotes the favorable uptake of pDNA into tumors.

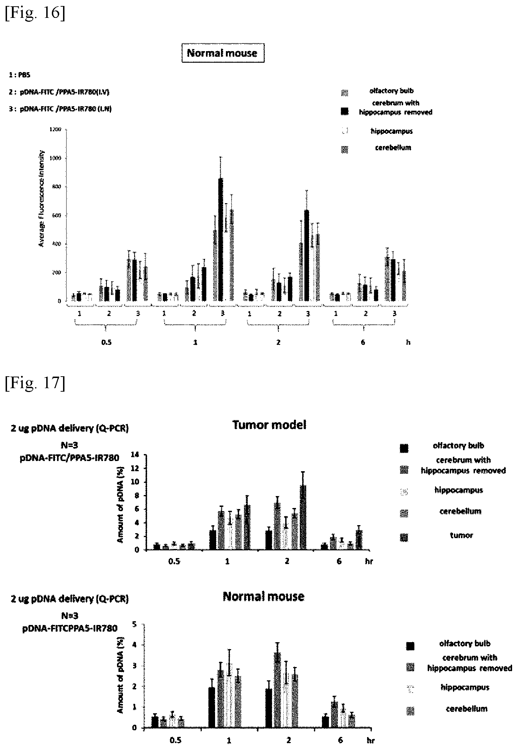

In a normal mouse model, the intranasal administration of pDNA-FITC/PPA5-IR780 led to efficient uptake of pDNA into brain tissue, similar to that observed in tumor-bearing mice in FIG. 15 (FIG. 16); Similar biodistribution patterns were observed in both mice, but the total amount of pDNA was little lower in the normal mice, compared with the tumor-bearing mice (FIG. 17). These results suggest that the presence of tumors is not necessary for efficient brain delivery of pDNA by PPA5-IR780.

To assess which component of the PPA5-IR780 polymer is critical for efficient brain uptake through an intranasal route, several polymer derivatives of PPA5-IR780 without either of pH-responsive or IR780 moieties were produced. To produce derivatives without pH-responsiveness, piperazine, which is a pH-responsive moiety, was substituted with dithiothreitol (DTT), which is a pH-responsive moiety, thereby producing P(DTT)PA5-IR780. In addition, control polymers having no photosensitizer for PPA5-IR780 and P(DTT)PA5-IR780 were produced, resulting in PPA5 and P(DTT)PA5 polymers, respectively. To compare the brain delivery efficiency of PPA5-IR780 and its derivatives without one or two functional moieties, pDNA-FITC was complexed with a P(DTT)PA5, P(DTT)PA5-IR780, PPA5 or PPA5-IR780 polymer and then administered via an intravenous route. At 0.5, 1, 2 and 6 hours after injection, various regions of the brain tissue were tested to confirm the presence of pDNA using a fluorescent reader.

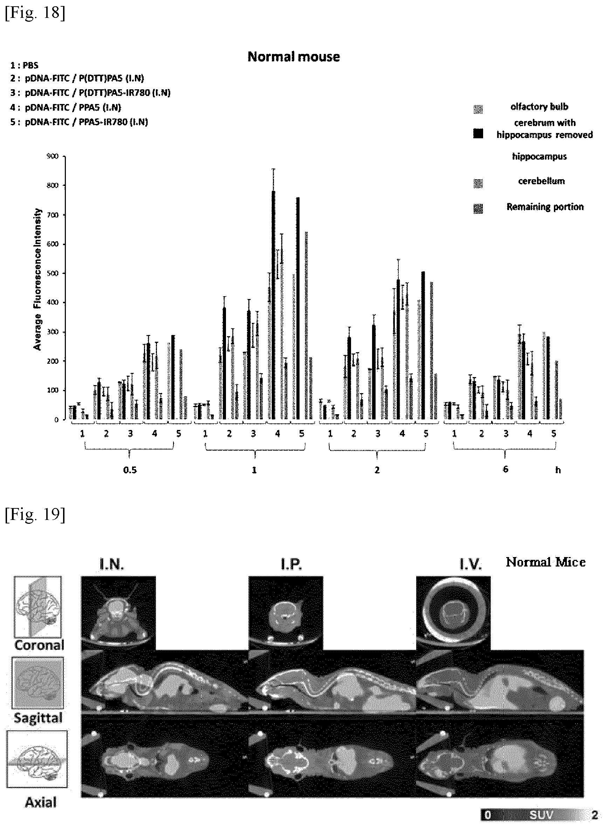

As shown in FIG. 18, FITC intensity was higher in mice treated with pH-responsive polymer-complexed pDNA-FITC (Groups 4 and 5), compared with groups treated with non-pH-responsive polymer-complexed pDNA-FITC (Groups 2 and 3). In addition, these results suggest that the brain uptake efficiency of an intranasally-administered pDNA/polymer complex did not depend on a photosensitizing IR780 moiety as Groups 2 and 4 showed accumulations in various regions, similar to Groups 3 and 5. Therefore, these results suggest that pH sensitivity is critical for efficient polymer-mediated delivery of a therapeutic agent through an intranasal route.

### <Experimental Example 11> Efficient delivery of oAd/PPA-Cu-64-DOTA-Herceptin complex by intranasal administration: PET/CT imaging

The brain delivery efficiency of the oAd/PPA-Cu-64-DOTA-Herceptin complex via intranasal administration, intraperitoneal administration and intravenous administration was measured using PET/CT imaging.

As shown in FIG. 19, at one hour after injection, compared with other delivery methods, the intranasal delivery exhibited the strongest signal in a brain region.

### <Experimental Example 12> PPA-Erbitux delivery in normal mice

By an LC-MS/MS experiment, the efficiencies of delivering therapeutic antibody-conjugated PPA to the brain via intravenous administration and intranasal administration were compared.

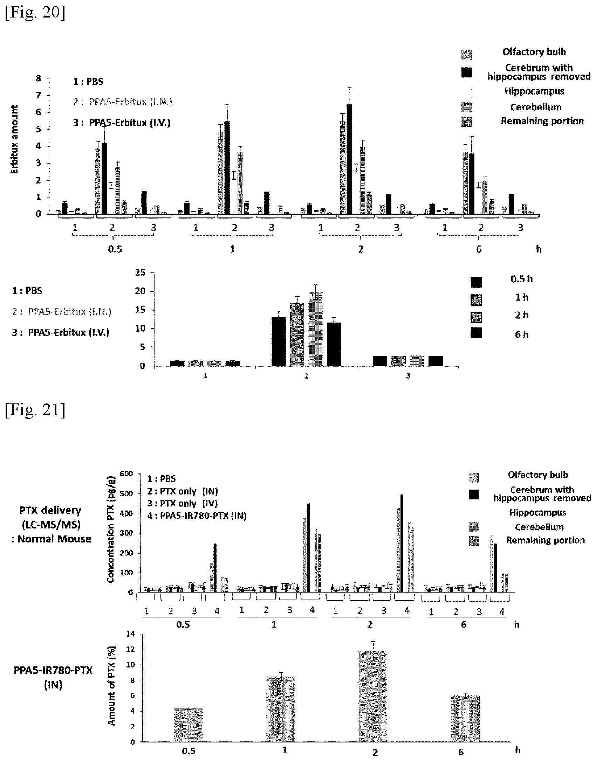

As shown in FIG. 20, the therapeutic antibody was capable of being effectively delivered to the brain due to PPA conjugation in an intranasally-administered experimental group, compared with comparative groups such as PBS or delivery via intravenous administration (IV). In addition, as a result of comparing administration efficiency over time in brain tissue, 2 hours after administration, the efficiency was detected at the highest level, which is 20% of the initial dose.

### <Experimental Example 13> Bioavailability of PPA-IR780-PTX after intranasal administration

An LC-MS/MS experiment was performed to compare the brain delivery efficiency of PPA-IR780 conjugated with PTX, which is a therapeutic drug, via intravenous administration and intranasal administration.

As shown in FIG. 21, PTX was successfully delivered to various brain regions through intravenous delivery. In addition, in brain tissue, PTX was detected at a high ratio, for example, up to 12% of the initial dose.

### <Experimental Example 14> Potent antitumor efficacy of PPA-PTX: MR imaging

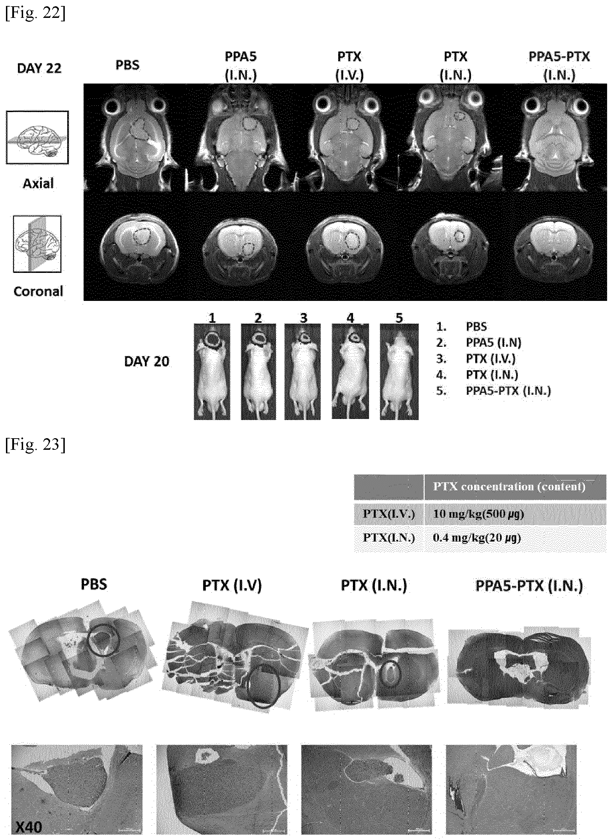

As shown in FIG. 22, MR imaging showed that the intranasal delivery of PPA5-PTX led to much greater antitumor efficacy than PTX I.V delivery and PTX I.N. delivery, and PPA5-PTX (I.N.) induced complete regression of brain tumors.

In addition, in a U87MG/Fluc orthotopic brain tumor model, at 13 days after injection, H&E staining showed that complete regression of a brain tumor was induced with a 1/25 dose of the PTX concentration (I.N. delivery) (FIG. 23).

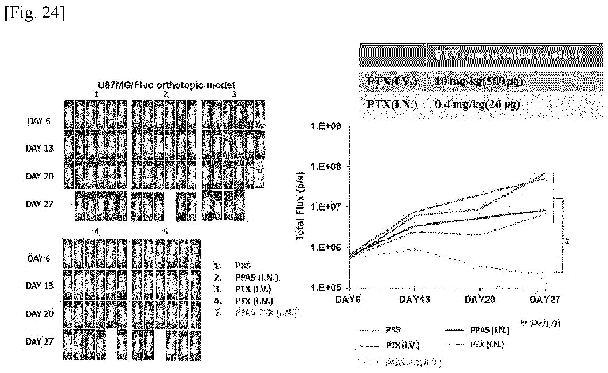

In addition, as shown in FIG. 24, it was shown that, in the U87MG/Fluc orthotopic brain tumor model, the intranasal delivery of PPA5-PTX led to much greater antitumor efficacy than those exhibited by PTX I.V. delivery and PTX I.N. delivery, and a 1/25 dose of the PTX concentration (I.N. delivery) led to much greater efficacy than intravenous delivery.

### [Industrial Applicability]

The present invention can be applied in diagnosis and treatment of a central nervous system disease, a neurodegenerative disease or a brain tumor.

## Claims

1. A polymer represented by Formula 1 below. 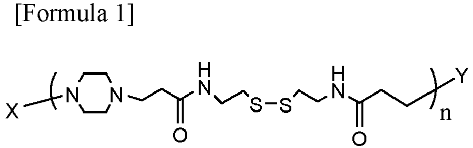 Wherein, X is PEG having a molecular weight of 2,000-10,000 Da Y is 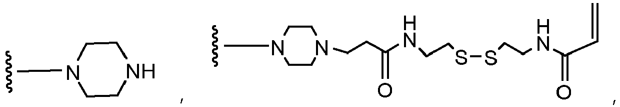 or arginine-conjugated PEI (polyethyleneimine), wherein PEI is branched PEI. n is 1 to 10.

2. A drug delivery system, comprising:
a polymer represented by Formula 1 below; and
one or more drugs selected from the group consisting of a therapeutic agent and a diagnostic agent: 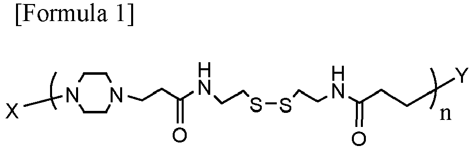 Wherein, X is PEG having a molecular weight of 2,000-10,000 Da
Y is 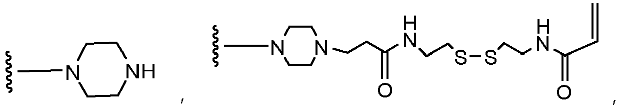 or arginine-conjugated PEI (polyethyleneimine), wherein PEI is branched PEI.
n is 1 to 10.

3. The drug delivery system according to claim 2, wherein the drug delivery system is used for intranasal administration.

4. The drug delivery system according to claim 2, wherein the polymer and the drug are complexed.

5. The drug delivery system according to claim 2, wherein the therapeutic agent is one or more selected form the group consisting of a gene delivery system, a photosensitizer and a pharmaceutically active component.

6. The drug delivery system according to claim 5, wherein the gene delivery system is (i) a naked recombinant DNA molecule, (ii) a plasmid, (iii) a viral vector, and (iv) a liposome or noisome containing the naked recombinant DNA molecule or plasmid.

7. The drug delivery system according to claim 6, wherein the viral vector is one or more selected from the group consisting of recombinant adenoviruses, adeno-associated viruses (AAVs), retroviruses, lentiviruses, herpes simplex viruses, vaccinia viruses, reoviruses, poxviruses, the Semliki forest virus and the measles virus.

8. The drug delivery system according to claim 5, wherein the photosensitizer is one or more selected from the group consisting of phorphyrins, chlorins, bacteriochlorins, phthalocyanines, naphthalocyanines and 5-aminolevuline esters.

9. The drug delivery system according to claim 5, wherein the pharmaceutically active component is one or more selected from the group consisting of an anticancer agent, an antibiotic, a hormone, a hormone antagonist, an interleukin, an interferon, a growth factor, a tumor necrosis factor, an endotoxin, a lymphotoxin, a urokinase, a streptokinase, a tissue plasminogen activator, a protease inhibitor, an alkylphosphocholine, a radioisotope-labeling component, a surfactant, a cardiovascular drug and a nervous system drug.

10. The drug delivery system according to claim 9, wherein the anticancer agent is one or more selected from the group consisting of bevacizumab, temozolomide, nitrosourea, cisplatin, procarbazine+CCNU+vincristine (PCV), vinblastine, vincristine, vinflunine, vindesine, vinorelbine, cabazitaxel, docetaxel, larotaxel, ortataxel, paclitaxel, tesetaxel, ixabepilone, herceptin, erbitux, cyclophosphamide, doxorubicin, etoposide, topotecan, carboplatin, procarbazine, mechlorethamine, ifosfamide, melphalan, chlorambucil, bisulfan, dactinomycin, daunorubicin, bleomycin, plicomycin, mitomycin, tamoxifen, transplatinum, 5-fluorouracil, and methotrexate.

11. The drug delivery system according to claim 2, wherein the drug delivery system is used in prevention or treatment of any one of a central nervous system disease, a neurodegenerative disease and a brain tumor.

12. The drug delivery system according to claim 11, wherein the central nervous system disease is any one selected from the group consisting of a cognitive disorder, intellectual disability, microencephaly, brain metastasis, a neurodevelopmental disorder, dementia, an autistic spectrum disorder, Down's syndrome, Rett syndrome, or fragile X syndrome.

13. The drug delivery system according to claim 11, wherein the neurodegenerative disease is any one selected from the group consisting of an ischemic stroke, a traumatic brain injury, acute disseminated encephalomyelitis, amyotrophic lateral sclerosis (ALS), retinitis pigmentosa, mild cognitive impairment, Alzheimer's disease, Pick's disease, senile dementia, progressive supranuclear palsy, cortical dementia, Wilson's disease, a multiple infarct disease, arteriosclerotic dementia, AIDS-associated dementia, cerebellar degeneration, spinocerebellar degeneration syndromes, Friedreich's ataxia, ataxia telangiectasia, an epilepsy-associated brain injury, a spinal cord injury, restless legs syndrome, Huntington's disease, Parkinson's disease, striatonigral degeneration, cerebral vasculitis, mitochondrial encephalomyopathies, neuronal ceroid lipofuscinosis, spinal muscular atrophies, a central nervous system-associated lysosomal storage disorder, leukodystrophies, a urea cycle defect disorder, hepatic encephalopathies, renal encephalopathies, metabolic encephalopathies, porphyria, bacterial meningitis, viral meningitis, meningoencephalitis, prion diseases, poisonings with neurotoxic compounds, Guillain Barre syndrome, chronic inflammatory neuropathies, polymyositis, dermatomyositis, and radiation-induced brain damage.

14. The drug delivery system according to claim 11, wherein the brain tumor is glioma, oligodendroglioma, glioblastoma, colloid cyst, epidermoid cyst, meningioma, hemangioblastoma, lymphoma, pituitary adenoma, a metastatic tumor, or a combination thereof.

15. The drug delivery system according to claim 2, wherein the diagnostic agent is a near-infrared fluorescent material, a radiopharmaceutical, or a contrast.

16. A composition for drug delivery to the brain through nasal route comprising a polymer represented by Formula 1 below as a carrier for drug delivery: 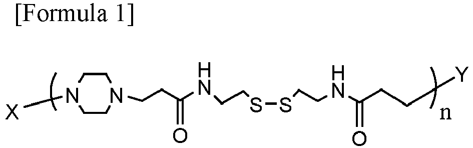 Wherein, X is PEG having a molecular weight of 2,000-10,000 Da Y is 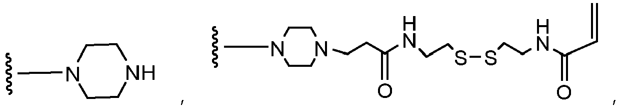 or arginine-conjugated PEI (polyethyleneimine), wherein PEI is branched PEI. n is 1 to 10.

17. A method of delivering a drug to the brain through nasal route comprising intranasally administering the drug delivery system of claim 2 to a subject.

18. A method of treating any one of a central nervous system disease, a neurodegenerative disease and a brain tumor, which comprises intranasally administering a pharmaceutically effective amount of the drug delivery system of claim 2 to a subject in need thereof.
